# EUROPÄISCHE PATENTANMELDUNG

(11) **EP 3 628 338 A1**
(43) Veröffentlichungstag der Anmeldung: **01.04.2020**
(21) Anmeldenummer: 19180940.9
(22) Anmeldetag: 18.06.2019
(51) Int. Cl.: A61L 2/10

(54) **VORRICHTUNG ZUM ENTKEIMEN VON MEHRWEG-BEHÄLTERN**

(30) Priorität: 31.08.2018 DE 102018121285; 28.02.2019 DE 102019105075; 05.03.2019 DE 102019105580
(71) Anmelder: Hilberer, Franz, 78234 Engen (DE)
(72) Erfinder: Hilberer, Franz, 78234 Engen (DE)
(74) Vertreter: Patentanwälte und Rechtsanwalt Weiß, Arat & Partner mbB

(57) **Zusammenfassung**

Die Erfindung betrifft eine Vorrichtung (V) zum Entkeimen von Mehrweg-Behältern (2, 3) mit einem Gehäuse (1), wobei in dem Gehäuse (1) eine Transporteinrichtung (4) vorhanden ist, wobei das Gehäuse (1) eine erste Öffnung (5) und eine zweite Öffnung (6) aufweist, wobei in dem Gehäuse (1) ein UV-C-Desinfizierer (7) vorhanden ist.

## Beschreibung

### Technisches Gebiet

Die Erfindung betrifft eine Vorrichtung zum Entkeimen von Mehrweg-Behältern nach dem Oberbegriff des Anspruchs 1.

### Stand der Technik

Derartige Vorrichtungen sind nicht bekannt. Im Einzelhandel werden vereinzelt Mehrweg-Behälter akzeptiert, selbst mit Wasser und Reinigungsmittel gereinigt, wobei dies aber gegen geltende Hygienevorschriften verstößt.

### Aufgabe der Erfindung

Die Aufgabe der vorliegenden Erfindung ist es, eine Vorrichtung zum Entkeimen von Mehrweg-Behältern bereitzustellen, welcher unter Einhaltung etwaiger Hygienevorschriften die Nutzung von Mehrweg-Behälter des Kunden nutzbar zu machen.

### Lösung der Aufgabe

Zur Lösung der Aufgabe führen die Merkmale nach dem Anspruch 1.

Vorteilhafte Ausgestaltungen sind in den Unteransprüchen beschrieben.

Eine erfindungsgemässe Vorrichtung zum Entkeimen von Mehrweg-Behältern weist ein Gehäuse auf, wobei in dem Gehäuse eine Transporteinrichtung vorhanden ist, wobei das Gehäuse eine erste Öffnung und eine zweite Öffnung aufweist, wobei in dem Gehäuse ein UVC-Desinfizierer vorhanden ist. Das Gehäuse kann hierbei aus einem metallenen Werkstoff oder Kunststoff hergestellt sein. Der UVC-Desinfizierer dient dabei einer schnellen vollständigen und praktischen Entkeimung der Mehrweg-Behälter.

Der UVC-Desinfizierer ist in der Regel eine UV-C - Lichtquelle. Bei UV-C - Lichtquelle kann es sich um eine Leuchtstoffröhre oder einer Birne oder auch um eine LED-Lampe handeln. Die LED-Lampe hätte den Vorteil, dass sie energiesparend wäre und sehr viel langlebiger als die üblichen UV-C - Birnen bzw. UV-C - Glühbirnen. Gerade vor dem Hintergrund, dass in der Vorrichtung der UVC-Desinfizierer nach jedem Durchlauf eines Mehrweg-Lebensmittelbehältnisses zur Entnahme ausgeschaltet und anschließend nach Eingabe eines weiteren Mehrweg-Lebensmittelbehältnisses wieder eingeschaltet wird.

Der UVC-Desinifizierer gibt ein spezielles UVC Licht (also Ultraviolettes Licht) ab. Mehrweg-Behälter, welche unter dem UVC-Desinfizierer durchläuft, wird dadurch mit UVC Licht der UVC - Desinfizierer bestrahlt und sorgt dabei zur Abtötung von Bakterien/Keime oder dergleichen.

Zum Einsparen von Einweg-Behältern, wie Plastiktüten nutzen die Kunden im Einzelhandel immer öfter Mehrweg-Behälter in Lebensmittelmärkten oder Metzgertheken oder anderen vergleichbaren Läden, in denen frische Lebensmittel verkauft werden. Dazu bringt der Kunde seinen eigenen Mehrweg-Behälter zum Befüllen der Frisch-Ware mit, um sie an den Bedientheken füllen zu lassen.

Was auf vielen Wochenmärkten schon angewandt und bei den umweltfreundlichen Kunden auch gerne genutzt wird, ist für die Lebensmittelmärkte aus hygienischen Gründen bisher nicht zulässig.

Doch um auch dem Kunden des Lebensmittelmarktes diese Möglichkeit zu geben ist hier eine erfindungsgemässe Vorrichtung gezeigt, bei der der Kunde seinen mitgebrachten Mehrweg-Behälter von der Kundenseite über eine erste Klappe in einen beispielsweise unter der Theken-Auslage eingebauten absolut dicht verschweißten Gehäuse als ein Übergabetunnel an den Verkäufer weiter.

Weiter ist vorgesehen, dass ein Boden des Übergabetunnels einen zweiten UVC-Desinfizierer umfasst. Dazu kann der zweite UVC-Desinfizierer in den Boden eingelassen oder auf dem Boden angeordnet sein.

In einem bevorzugten Ausführungsbeispiel ist neben der Transporteinrichtung eine zweite Transporteinrichtung vorhanden, wobei die beiden Transporteinrichtungen in Linie angeordnet sind und zwischen der ersten Transporteinrichtung und der zweiten Transporteinrichtung ein Freiraum angeordnet ist, wobei die erste Transporteinrichtung und die zweite Transporteinrichtung sich von der ersten Öffnung zu der zweiten Öffnung erstrecken. Dabei darf der Freiraum zwischen den beiden Transporteinrichtungen nicht so groß sein, alsdass Mehrweg-Behälter oder deren Behälterdeckel in den Freiraum fallen oder sich dort verkeilen können.

Im Bereich des Freiraums kann der zweite UVC-Desinfizierer angeordnet sein. Der Freiraum darf daher nicht so klein sein, alsdass der zweite UVC-Desinfizierer keine ausreichende Gelegenheit seine desinfizierende Wirkung zu entfalten. In der Regel beträgt der Abstand zwischen den beiden Transportvorrichtungen, also der Freiraum 3- 20 cm und kann in Einzelfällen auch von dieser Maßgabe abweichen.

Auf diese Weise können abgenommene und zu den Transporteinrichtungen hin gelegte Behälterdeckel der Mehrweg-Behälter von unten im Bereich des Freiraums durch den zweiten UVC-Desinfizierer angestrahlt und desinfiziert werden.

Außerdem können auch weitere UVC-Desinfizierer vorgesehen sein, die an den Seitenwandungen des Übergabetunnels angeordnet sind. Als Seitenwände gelten die Wände des Übergabetunnels, die nicht Boden oder Decke sind.

Sobald der Übergabetunnel auf beiden Seiten geschlossen ist, wird der Mehrweg-Behälter durch den in dem Übergabetunnel integrierte UVC-Desinfizierer entkeimt. Der UVC-Desinfizierer wird aus Sicherheitsgründen über einen Druckschalter an der Bedienseite so geregelt, dass sie nur aktiviert werden kann, wenn beide Klappen dicht geschlossen sind.

Das Innere des Gehäuses kann außerdem eine reflektierende Oberfläche aufweisen. Auf die Weise können die Strahlen des oder der UVC-Desinfizierer gespiegelt und verstärkt werden, um eine bessere Desinfektionsleistung zu erreichen.

Das Bedienungspersonal entnimmt auf der Bedienseite den keimfreien Mehrweg-Behälter des Kunden und befüllt ihn mit der Frischware.

Der Transport der Mehrweg-Behälter im Übergabetunnel erfolgt beispielsweise durch einen elektrischen Rohrmotor, der ein Transport- oder Förderband antreibt. Die Motorlaufrichtung ist reversibel, so dass theoretisch auch eine Rückgabe des Behälters über den Übergabetunnel erfolgen könnte.

Die gesamte elektrische Einrichtung ist nach DIN ELT IP ausgeführt. Die Vorrichtung kann sowohl in Bedientheken bzw. Ladentheken integriert werden oder auch als sogenanntes Stand-Alone-Gerät zwischengebaut oder angebaut werden.

Die Vorrichtung kann aus Edelstahl der Materialgüte 18/10 hergestellt werden und ist innen absolut glatt und dicht verschweißt, und lässt sich für Revisions- und Reinigungsarbeiten komplett herausnehmen. Durch seine Bauart kann das Gehäuse so überall eingesetzt werden wo es benötigt wird, auch in gekühlten Bereichen. In diesem Fall wird die Vorrichtung doppelwandig mit 20mm Isolierung ausgeführt.

Die Größe der Vorrichtung und das äußere Design lassen sich dem Kundenwunsch anpassen.

Die Vorrichtung weist also die Transporteinrichtung auf, welche sich von der ersten Öffnung zu der zweiten Öffnung erstreckt. Die erste Öffnung weist die erste Klappe und die zweite Öffnung die zweite Klappe (9) auf.

Weiter weist die erste Klappe und/oder die erste Öffnung einen ersten Sensor auf. Die zweite Klappe und/oder die zweite Öffnung weist wiederum einen zweiten Sensor auf. Es handelt sich dabei um eine Sicherungseinrichtung. Dadurch soll erreicht werden, dass der UVC-Desinfizierer nur bei geschlossenen Klappen in Betrieb genommen werden kann. Dazu ist der UVC-Desinfizierer mit dem ersten und dem zweiten Sensor wirkverbunden.

Die Transporteinrichtung ist dazu an einem Boden des Gehäuses angeordnet und der UVC-Desinfizierer ist an einer Decke des Gehäuses angeordnet, wobei der UVC-Desinfizierer von der Decke zu dem Boden hin ausgerichtet ist.

Die erste Öffnung weist weiter einen ersten Auffangwinkel auf und die zweite Öffnung weist einen zweiten Auffangwinkel auf, wobei die Transporteinrichtung zwischen dem ersten Auffangwinkel und dem zweiten Auffangwinkel angeordnet ist. Die Auffangwinkel sind dann vorteilhaft, wenn die Transporteinrichtung sich nicht direkt bis zu den Klappen erstrecken soll und der Mehrweg-Behälter bei der Klappe ankommt, dieser nicht gegen die Klappe stoßen kann, sondern im Auffangwinkel zum Stehen kommt.

Weiter weist das Gehäuse eine Kühlluftzuleitung auf. Dies ist besonders dann vorteilhaft, wenn die Vorrichtung und das Gehäuse als Teil einer Ladentheke ausgebildet sind und die Kühlluft aus der Ladentheke in das Gehäuse geblasen werden kann. Dadurch wird die Kühlkette möglichst lange aufrechterhalten.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
- Figur 1: eine geschnittene Seitenansicht einer erfindungsgemässen Vorrichtung V;
- Figur 2: eine erste Ansicht einer Ladentheke (L) und einer weiteren erfindungsgemäßen Vorrichtung V;
- Figur 3: eine Draufsicht auf die Ladentheke (L) aus Figur 2;
- Figur 4: eine geschnittene Seitenansicht der Ladentheke (L) aus den Figuren 2 und 3.

### Ausführungsbeispiel

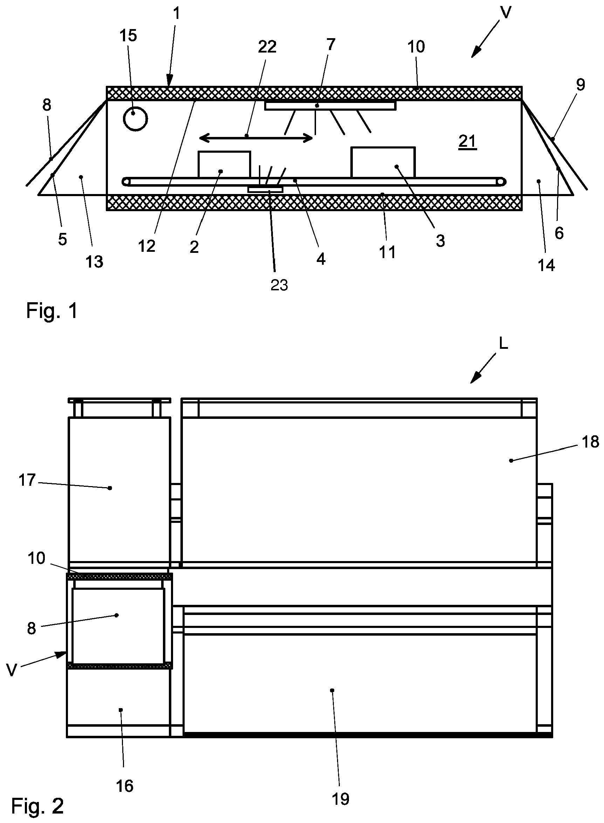

In Figur 1 ist eine geschnittene Seitenansicht einer erfindungsgemässen Vorrichtung V gezeigt. Dort ist eine Gehäuse 1 gezeigt. Das Gehäuse 1 besteht aus einem Boden 11, einer Decke 12 und zwei nicht näher beschriebene Seitenwände, welche einen Übergabetunnel 21 umschließen. Weiter weist der Übergabetunnel 21 weist einends eine erste Öffnung 5 und andernends eine zweite Öffnung 6 auf.

Die erste Öffnung 5 weist eine erste Klappe 8 auf. Die zweite Öffnung 6 weist eine zweite Klappe 9 auf. In der hier gezeigten Position sind beide Klappen 8, 9 leicht geöffnet dargestellt. Weiter ist in dem Übergabetunnel 21 eine Transporteinrichtung 4 gezeigt. Dabei kann es sich um ein Walzenbett oder ein Umlaufband handeln.

Die Transporteinrichtung 4 ist an dem Boden 11 angeordnet und erstreckt sich von einem ersten Auffangwinkel 13 im Bereich der ersten Öffnung 5 zu einem zweiten Auffangwinkel 14 im Bereich der zweiten Öffnung 6.

Auf der Transporteinrichtung 4 sind zwei Mehrweg-Behälter 2, 3 gezeigt. Diese beiden Mehrweg-Behälter 2, 3 können auf der Transporteinrichtung 4 zu der ersten Öffnung 5 oder zu der zweiten Öffnung 6 bewegbar sind. Dies ist durch einen Bewegungspfeil 22 angedeutet.

Weiter ist in dem Gehäuse 1 ein UVC-Desinfizierer 7 vorhanden. Der UVC-Desinfizierer 7 ist an der Decke 12 angeordnet. Dabei ist der UVC-Desinfizierer 7 von der Decke 12 hin zu dem Boden 11 ausgerichtet.

Außerdem umfasst der Boden 11 in diesem Ausführungsbeispiel einen zweiten UVC-Desinfizierer 23. Dieser strahlt von dem Boden 11 weg zu der Decke 12. Dabei ist die Transportvorrichtung 4 beispielsweise transparent ausgeführt.

Ausserdem umgibt das Gehäuse 1 eine Isolierung 10. Die Isolierung 10 kann beispielsweise Polystyrol sein.

Weiter umfasst die erste Klappe 8 und/oder die erste Öffnung 5 einen ersten Sensor. In gleicher Weise umfasst auch die zweite Klappe 9 und/oder die zweite Öffnung 6 einen zweiten Sensor. Der UVC-Desinfizierer 7 und auch der zweite UVC-Desinfizierer 23 sind mit dem ersten und dem zweiten Sensor wirkverbunden. Das bedeutet im Einzelnen, dass der UVC-Desinfizierer 7 und der zweite UVC-Desinfizierer 23 nur dann strahlt ist, wenn beide Klappen 8, 9 die beiden Öffnungen 5, 6 durch die beiden Sensoren als geschlossen detektiert sind.

Weiter weist die erste Öffnung 5 einen ersten Auffangwinkel 13 auf und die zweite Öffnung 6 einen zweiten Auffangwinkel 14, wobei die Transporteinrichtung 4 zwischen dem ersten Auffangwinkel 13 und dem zweiten Auffangwinkel 14 angeordnet ist.

Zuletzt weist das Gehäuse 1 eine Kühlluftzuleitung 15 auf. Dazu kann von einer Ladentheke L gekühlte Luft in den Übergabetunnel 21 eingeleitet werden, um die Kühlkette möglichst lange aufrecht zu erhalten.

In Figur 2 ist eine Frontansicht einer Ladentheke L von Kundenseite aus gezeigt. Hier ist gezeigt, wie die erfindungsgemässe Vorrichtung V als Teil einer Ladentheke ausgebildet ist. In der Frontansicht der Figur 2 ist eine erste Klappe 8 der Vorrichtung V gezeigt. Ausserdem ist die Isolierung 10 gezeigt. Unterhalb der Vorrichtung V ist ein Bodenabschluss 16 gezeigt. Ausserdem ist eine Obertheke 17 gezeigt, die oberhalb der Vorrichtung V angeordnet ist.

Neben der Vorrichtung V und dem Bodenabschluss 16 ist ein Thekenelement 19 zu erkennen. Oberhalb ist ein Spuckschutz 18 angeordnet.

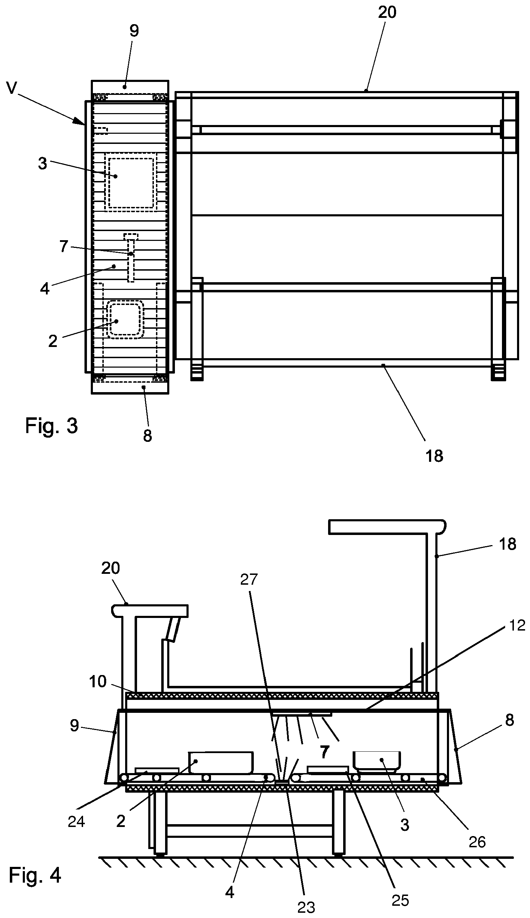

In Figur 3 ist eine geschnittene Draufsicht der Ladentheke L aus Figur 2 gezeigt. Neben der ersten Klappe 8 auf Kundenseite ist hier auch die zweite Klappe 9 auf Bedienpersonalseite gezeigt. Weiter ist der UVC-Desinfizierer 7 im Übergabetunnel 21 gezeigt. Ausserdem ist zu erkennen, wie die Mehrweg-Behälter 2, 3 auf der Transporteinrichtung 4 stehen. Ausserdem sind eine Bedienpersonaltheke 20 und der Spuckschutz 18 gezeigt.

In Figur 4 ist eine geschnittene Seitenansicht der Ladentheke L aus den Figuren 2 und 3 gezeigt. Dort sind die bereits vorher beschriebenen technischen Merkmale der Vorrichtung V für sich und als Teil einer Ladentheke L gezeigt. Um Wiederholungen zu vermeiden wird darauf abgestellt, dass die bereits für die vorigen Figuren getroffenen Feststellungen auch für die Figur 4 gelten sollen. Allerdings ist in der Figur 4 nicht nur die eine Transporteinrichtung 4, sondern auch eine in Linie angeordnete zweite Transporteinrichtung 26 gezeigt. Zwischen der ersten Transporteinrichtung 4 und der zweiten Transporteinrichtung 23 ist ein Freiraum 27 vorhanden. Außerdem sind neben den Mehrweg-Behältern 2, 3 noch dazugehörige Behälterdeckel 24, 25 gezeigt. Die Behälterdeckel 24, 25 können dabei mit ihrer Abdeckinnenseite zu den beiden Transporteinrichtungen 4, 26 oder zu der Decke 12 hin angeordnet sein. Je nachdem, wie sie angeordnet sind, werden sie von dem UVC-Desinifizierer 7 oder dem zweiten UVC-Desinfizierer 23 sterilisiert.

Bezugnehmend auf die Figuren 1 bis 4 erklärt sich die Funktionsweise der erfindungsgemässen Vorrichtung V in folgenden Schritten:

### 1. Schritt:

Ein Kunde öffnet die erste Klappe 8 und legt seinen mitgebrachten Mehrweg-Behälter 2, 3 und die dazugehörigen Behälterdeckel 24, 25 getrennt von den Mehrweg-Behältern 2, 3 auf die Transporteinrichtung 4.

### 2. Schritt:

Der Kunde schließt die erste Klappe 8. Das Bedienpersonal setzt durch einen nicht näher beschriebenen Druckschalter die Transporteinrichtung 4 in Bewegung, wobei auch eine automatische Inbetriebnahme bei Schliessen beider Klappen 8, 9 möglich sein soll. Dabei wird gleichzeitig der UVC-Desinfizierer 7 und/oder der zweite UVC-Desinfizierer 23 eingeschaltet, wobei der UVC-Desinfizierer 7 und/oder der zweite UVC-Desinfizierer 23 nur eingeschaltet wird, wenn die beiden Sensoren detektieren, dass die beiden Öffnungen 5, 6 durch die beiden Klappen 8, 9 verschlossen sind.

### 3. Schritt:

Die Mehrweg-Behälter 2, 3 laufen bei eingeschaltetem UVC-Desinfizierer 7 und/oder der zweite UVC-Desinfizierer 23 weiter auf der Transporteinrichtung 4 bzw. der zweiten Transporteinrichtung 26 hin zu der der zweiten Öffnung 6.

### 4. Schritt:

Das Bedienpersonal stoppt mit dem Druckschalter die Transporteinrichtung 4 und deaktiviert den UVC-Desinfizierer 7 und/oder den zweiten UVC-Desinfizierer 23.

### 5. Schritt:

Das Bedienpersonal öffnet die zweite Klappe 9 und entnimmt die Mehrweg-Behälter 2, 3 von der Transporteinrichtung 4 und befüllt die Mehrweg-Behälter 2, 3 mit den Bestellungen des Kunden.

**Bezugszeichenliste**

| | |
|---|---|
| 1 | Gehäuse |
| 2 | Mehrweg-Behälter |
| 3 | Mehrweg-Behälter |
| 4 | Transporteinrichtung |
| 5 | Erste Öffnung |
| 6 | Zweite Öffnung |
| 7 | UVC-Desinfizierer |
| 8 | Erste Klappe |
| 9 | Zweite Klappe |
| 10 | Isolierung |
| 11 | Boden |
| 12 | Decke |
| 13 | Erster Auffangwinkel |
| 14 | Zweiter Auffangwinkel |
| 15 | Kühlluftzuleitung |
| 16 | Bodenabschluss |
| 17 | Obertheke |
| 18 | Spuckschutz |
| 19 | Thekenelement |
| 20 | Personaltheke |
| 21 | Übergabetunnel |
| 22 | Bewegungspfeil |
| 23 | Weiterer UVC-Desinfizierer |
| 24 | Behälterdeckel |
| 25 | Behälterdeckel |
| 26 | Zweite Transporteinrichtung |
| 27 | Freiraum |
| | |
| | |
| V | Vorrichtung |
| L | Ladentheke |

## Patentansprüche

1. Vorrichtung (V) zum Entkeimen von Mehrweg-Behältern (2, 3) mit einem Gehäuse (1), wobei in dem Gehäuse (1) eine Transporteinrichtung (4) vorhanden ist, wobei das Gehäuse (1) eine erste Öffnung (5) und eine zweite Öffnung (6) aufweist,
**dadurch gekennzeichnet**,
in dem Gehäuse (1) ein UVC-Desinfizierer (7) vorhanden ist.

2. Vorrichtung nach Anspruch 1, **dadurch gekennzeichnet, dass** ein Boden (11) einen zweiten UVC-Desinfizierer (23) umfasst.

3. Vorrichtung nach Anspruch 1 oder 2, **dadurch gekennzeichnet, dass** sich die Transporteinrichtung (4) von der ersten Öffnung (5) zu der zweiten Öffnung (6) erstreckt.

4. Vorrichtung nach Anspruch 1, **dadurch gekennzeichnet, dass** neben der Transporteinrichtung (4) eine zweite Transporteinrichtung (26) vorhanden ist, wobei die beiden Transporteinrichtungen in Linie angeordnet sind und zwischen der ersten Transporteinrichtung (5) und der zweiten Transporteinrichtung (26) ein Freiraum (27) angeordnet ist, wobei die erste Transporteinrichtung (4) und die zweite Transporteinrichtung (26) sich von der ersten Öffnung (5) zu der zweiten Öffnung (6) erstrecken.

5. Vorrichtung nach Anspruch 4, **dadurch gekennzeichnet, dass** im Bereich des Freiraums (27) der zweite UVC-Desinfizierer (23) angeordnet ist.

6. Vorrichtung nach einem der vorigen Ansprüche, **dadurch gekennzeichnet, dass** die erste Öffnung (5) eine erste Klappe (8) und die zweite Öffnung (6) eine zweite Klappe (9) aufweist.

7. Vorrichtung nach einem der vorigen Ansprüche, **dadurch gekennzeichnet, dass** die erste Klappe (8) und/oder die erste Öffnung (5) einen ersten Sensor umfasst/en und die zweite Klappe (9) und/oder die zweite Öffnung (6) einen zweiten Sensor umfasst/en

8. Vorrichtung nach einem der vorigen Ansprüche, **dadurch gekennzeichnet, dass** das Innere des Gehäuses eine reflektierende Oberfläche aufweist.

9. Vorrichtung nach Anspruch 8, **dadurch gekennzeichnet, dass** der UVC-Desinfizierer (7) und/oder der zweite UVC-Desinfizierer (26) mit dem ersten und dem zweiten Sensor wirkverbunden ist/sind.

10. Vorrichtung nach einem der vorigen Ansprüche, **dadurch gekennzeichnet, dass** die Transporteinrichtung (4) beabstandet an einem Boden (11) des Gehäuses (1) angeordnet ist und der UVC-Desinfizierer (7) an einer Decke (12) des Gehäuses (1) angeordnet ist, wobei der UVC-Desinfizierer (7) von der Decke (12) zu dem Boden (11) ausgerichtet ist.

11. Vorrichtung nach einem der vorigen Ansprüche, **dadurch gekennzeichnet, dass** die erste Öffnung (5) einen ersten Auffangwinkel (13) aufweist und die zweite Öffnung (6) einen zweiten Auffangwinkel (14) aufweist, wobei die Transporteinrichtung (4) zwischen dem ersten Auffangwinkel (13) und dem zweiten Auffangwinkel (14) angeordnet ist.

12. Vorrichtung nach einem der vorigen Ansprüche, **dadurch gekennzeichnet, dass** das Gehäuse eine Kühlluftzuleitung (15) aufweist.

13. Vorrichtung nach einem der vorigen Ansprüche, **dadurch gekennzeichnet, dass** das Gehäuse (1) als Teil einer Ladentheke (L) ausgebildet ist.
